# EUROPEAN PATENT SPECIFICATION

(11) **EP 2 376 906 B1**
(45) Date of publication and mention of the grant of the patent: **01.01.2014**
(21) Application number: 08878455.8
(22) Date of filing: 28.11.2008
(51) Int. Cl.: G01N 30/90, G01N 33/53, G01N 33/48, G01N 33/487, G01N 33/558, G01N 33/58

(54) **METHOD FOR AMPLIFICATION OF SIGNAL IN IMMUNOCHROMATOGRAPHIC ASSAY AND IMMUNOCHROMATOGRAPHIC KIT USING THE METHOD**
VERFAHREN ZUR SIGNALVERSTÄRKUNG BEI EINEM IMMUNCHROMATOGRAPHIETEST SOWIE IMMUNCHROMATOGRAPHIE-KIT MIT VERWENDUNG DES VERFAHRENS
PROCÉDÉ D'AMPLIFICATION DE SIGNAL DANS UNE ANALYSE IMMUNOCHROMATOGRAPHIQUE ET KIT IMMUNOCHROMATOGRAPHIQUE UTILISANT LE PROCÉDÉ

(43) Date of publication of application: 19.10.2011
(73) Proprietor: Infopia Co., Ltd., Gyeonggi-do 431-080 (KR)
(72) Inventor: BAE, Byeong-Woo, Anyang Gyeonggi-do 431-080 (KR); LEE, Sung-Dong, Anyang Gyeonggi-do 431-760 (KR); KIM, Min-Gon, Daejeon 305-759 (KR); SHIN, Yong-Beom, Daejeon 302-772 (KR); JANG, Jin-Hee, Anyang Gyeonggi-do 431-807 (KR); SHIN, Ji-Hun, Seoul 150-840 (KR)
(74) Representative: Nederlandsch Octrooibureau
(86) International application number: PCT/KR2008/007049
(87) International publication number: WO 2010/061992

(56) References cited:
- EP-A1- 1 020 726
- WO-A2-2008/073222
- KR-A- 20050 058 154
- KR-A- 20090 011 820
- KR-B1- 100 809 377
- US-A- 5 968 839
- US-A1- 2005 112 780
- US-B2- 7 451 649
- SEYDACK ET AL: "Nanoparticle labels in immunosensing using optical detection methods", BIOSENSORS AND BIOELECTRONICS, ELSEVIER BV, NL, vol. 20, no. 12, 15 June 2005 (2005-06-15) , pages 2454-2469, XP027619312, ISSN: 0956-5663 [retrieved on 2005-06-15]
- LIU ET AL: "Nanomaterial labels in electrochemical immunosensors and immunoassays", TALANTA, ELSEVIER, AMSTERDAM, NL, vol. 74, no. 3, 16 October 2007 (2007-10-16), pages 308-317, XP022384822, ISSN: 0039-9140, DOI: 10.1016/J.TALANTA.2007.10.014

## Description

### Technical Field

The present invention relates to a method for amplifying a signal in an immunochromatographic assay for high-sensitive detection of an analyte and an immunochromatographic kit using the method, and more particularly, to a method for amplifying a signal in an immunochromatographic assay for high-sensitive detection of an analyte and an immunochromatographic kit using the method, which amplifies a signal by controlling a flow rate of indicators by discrimination between the size of a first indicator and the size of a second indicator binding to the first indicator in a sandwich assay method.

### Background Art

An immunochromatographic assay is a method that can test an analyte qualitatively and quantitatively within a short time by using the property that biological materials or chemical materials bind specifically to each other. Particularly, a sandwich-type immunoassay method is well known as immobilizing a first antibody, which is specific to a first epitope of an analyte to be tested for presence and concentration, to a solid support body and using a second antibody specific to a second epitope of the analyte.

An assay strip or an immunoassay kit, which is an assembly of the assay strip into a housing, is generally used as a kit for the immunochromatographic assay. When a fluid containing an analyte is applied to one side of a porous strip, the fluid flows due to a capillary phenomenon and an assay target binds to an immobilized antibody and an antibody containing an indicator, thus completing a sandwich-type assay method.

Generally, in an immunochromatographic kit, an antibody is immobilized to a membrane where a fluid sample can flow due to a capillary phenomenon, a sample pad and a conjugate pad are provided at the upstream side of the membrane, and an absorbing pad is connected to the downstream side of the membrane. The sample pad absorbs a liquid sample containing an analyte and provides a uniform flow, and an indicator, to which an antibody capable of binding selectively to the analyte is bound, is dried at the conjugate pad. An immobilized antibody binding selectively to an analyte and a material capable of binding to an antibody immobilized to an indicator are immobilized at the different positions of the membrane to form a detection site and a control site, respectively. An antibody immobilized to the membrane capable of binding selectively to the analyte and an antibody immobilized to the indicator may be configured to bind to the analyte in a sandwich type. The absorbing pad is formed of a material capable of absorbing a liquid sample. In the immunochromatographic kit, when a liquid sample containing an analyte is dropped on a sample pad, an indicator - antibody having a selectivity to the analyte and an antibody immobilized to a membrane bind to analyte in a sandwich type, thus forming a band that can be identified with the naked eye at the position of the membrane where the antibody is immobilized.

An immunochromatographic signal amplification method is disclosed in the related art that binds a primary conjugate body and an antigen, additionally binds a secondary conjugate body thereto, and they finally bind to an antibody immobilized to a membrane. However, because the detection sensitivity of the related art immunochromatographic method is low, it is difficult to detect a sample that requires a higher sensitivity.

Thus, the present inventors have completed the present invention by detecting the fact that the sensitivity of signal amplification increases when an antigen and a primary conjugate body bind to each other and they bind to an immobilized antibody in a sandwich reaction and then a secondary conjugate body binds thereto on an immunochromatography. In particular, the present invention performs signal amplification through one-time sample injection without separate mechanical control or artificial step-by-step reaction.

### Disclosure of Invention

### Technical Problem

An aspect of the present invention provides a method for amplifying a signal in an immunochromatographic assay.

Another aspect of the present invention provides an immunochromatographic kit with an increased sensitivity by using the signal amplification method.

### Technical Solution

According to an aspect of the present invention, there is provided a method for amplifying a signal in an immunochromatographic assay, including: binding a primary conjugate body, which has a first antibody binding specifically to a first epitope of an analyte, a connector, and a first indicator particle to which the first antibody and the connector are bound, to the analyte; binding the analyte bound to the primary conjugate body to an immobilized second antibody binding specifically to a second epitope of the analyte; and binding a secondary conjugate body, which has a third antibody binding specifically to the connector of the primary conjugate body and a second indicator particle to which the third antibody is bound, to the connector of the primary conjugate body, wherein the primary conjugate body is disposed nearer to the immobilized second antibody than the secondary conjugate body, and the particle of the first indicator has a size of about 10 nm to about 20 nm and the particle of the second indicator has a size of about 20 nm to about 60 nm wherein the particle of the second indicator is larger than the particle of the first indicator, so that the secondary conjugate body reaches the immobilized second antibody later than the primary conjugate body.

According to another aspect of the present invention, there is provided a method for amplifying a signal in an immunochromatographic assay, comprising: binding a primary conjugate body, which has a first antibody binding specifically to a first epitope of an analyte and a first indicator particle to which the first antibody is bound, to the analyte; binding the analyte bound to the primary conjugate body to an immobilized second antibody binding specifically to a second epitope of the analyte; and binding a secondary conjugate body, which has a third antibody binding specifically to the first antibody of the primary conjugate body and a second indicator particle to which the third antibody is bound, to the first antibody of the primary conjugate body, wherein the primary conjugate body is disposed nearer to the immobilized second antibody than the secondary conjugate body, and the particle of the first indicator has a size of about 10 nm to about 20 nm and the particle of the second indicator has a size of about 20 nm to about 60 nm wherein the particle of the second indicator is larger than the particle of the first indicator, so that the secondary conjugate body reaches the immobilized second antibody later than the primary conjugate body.

According to another aspect of the present invention, there is provided an immunochromatographic kit comprising:
a sample pad to which a liquid sample containing an analyte is applied;
a conjugate pad comprising a primary conjugate body having a first antibody binding specifically to a first epitope of an analyte, a connector, and a first indicator to which the first antibody and the connector are bound, and a secondary conjugate body having a third antibody binding specifically to the connector of the primary conjugate body and a second indicator to which the third antibody is bound, wherein the primary conjugate body is disposed nearer to an immobilized second antibody than the secondary conjugate body and the particle of the first indicator has a size of about 10 nm to about 20 nm and the particle of the second indicator has a size of about 20 nm to about 60 nm wherein the second indicator is larger than the first indicator so that the secondary conjugate body reaches the immobilized second antibody later than the primary conjugate body;
a membrane comprising a detection site immobilizing thereto the second antibody binding specifically to a second epitope of the analyte to which the primary conjugate body is bound, and a control site for error detection; and
an absorbing pad absorbing the liquid sample by a capillary phenomenon.

According to another aspect of the present invention, there is provided an immunochromatographic kit comprising:
a sample pad to which a liquid sample containing an analyte is applied;
a conjugate pad comprising a primary conjugate body having a first antibody binding specifically to a first epitope of an analyte and a first indicator to which the first antibody is bound, and a secondary conjugate body having a third antibody binding specifically to the first antibody of the primary conjugate body and a second indicator to which the third antibody is bound, wherein the primary conjugate body is disposed nearer to an immobilized second antibody than the secondary conjugate body and the particle of the first indicator has a size of about 10 nm to about 20 nm and the particle of the second indicator has a size of about 20 nm to about 60 nm wherein the second indicator is larger than the first indicator so that the secondary conjugate body reaches the immobilized second antibody later than the primary conjugate body;
a membrane comprising a detection site immobilizing thereto the second antibody binding specifically to a second epitope of the analyte to which the primary conjugate body is bound, and a control site for error detection; and
an absorbing pad absorbing the liquid sample by a capillary phenomenon.

### Advantageous Effects

The signal amplification method of the present invention immobilizes a primary conjugate body to a capture antibody together with an antigen and then binds a secondary conjugate body to the primary conjugate body, thereby providing a good signal amplification effect. Also, the use of the signal amplification method makes it possible to fabricate an immunochromatographic kit with an increased sensitivity.

### Brief Description of Drawings

FIG. 1 is a diagram illustrating a process of signal amplification in assaying an antigen by a method of the present invention, in which a reference numeral 1 denotes a first antibody, a reference numeral 2 denotes a connector, a reference numeral 3 denotes a first indicator, a reference numeral 4 denotes an analyte, a reference numeral 5 denotes a second antibody, a reference numeral 6 denotes a third antibody, a reference numeral 7 denotes a second indicator, a reference numeral 8 denotes a primary conjugate body, and a reference numeral 9 denotes a secondary conjugate body.

FIG. 2A is a diagram illustrating a structure of an immunochromatographic kit according to an embodiment of the present invention, in which a reference numeral 10 denotes a sample pad, a reference numeral 11 denotes a conjugate pad, a reference numeral 12 denotes a membrane, a reference numeral 13 denotes a detection site, a reference numeral 14 denotes a control site, a reference numeral 15 denotes an absorbing pad, a reference numeral 17 denotes a site immobilizing a secondary conjugate body, and a reference numeral 18 denotes a site immobilizing a primary conjugate body.

FIG. 2B is a diagram illustrating a structure of an immunochromatographic kit having two conjugate pads according to another embodiment of the present invention, in which a reference numeral 16 denotes a second conjugate pad, a reference numeral 17 denotes a site immobilizing a secondary conjugate body, and a reference numeral 19 denotes a first conjugate pad applied entirely with primary conjugate body.

FIG. 2C is a diagram illustrating an assembly structure of the immunochromatographic kit of FIG. 2B.

FIGS. 3A and 3B are graphs illustrating the result of a measurement of a signal amplification effect according to the size of a nano particle used in the secondary conjugate body, which is performed using a troponin I as an antigen.

FIG. 4A illustrates the result of an experiment on a signal amplification effect according to the separation of the conjugate bodies, which is performed using troponin I, and FIG. 4B illustrates a graph thereof.

FIG. 5A illustrates the result of an experiment on a signal amplification effect according to the separation of the conjugate bodies, which is performed using myoglobin, and FIG. 5B illustrates a graph thereof.

### Best Mode for Carrying out the Invention

Exemplary embodiments of the present invention will now be described in detail with reference to the accompanying drawings.

In this specification, the term 'connector' represents a material that binds specifically to a third antibody by an antigen-antibody reaction so that a secondary conjugate body can bind to a primary conjugate body. In the present invention, the primary conjugate body contains a first antibody, a connector, and a first indicator. However, if the first antibody can bind to a third antibody of a secondary conjugate body, the primary conjugate body may not contain the connector. Herein, the primary conjugate body is immobilized to a capture antibody while flowing through a strip after binding to an antigen. In the present invention, the secondary conjugate body contains a second indicator and a third antibody specific to the connector of the primary conjugate body. Herein, the third antibody binds specifically to a connector of the primary conjugate body to amplify a signal. However, if the third antibody can bind specifically to the first antibody and thus the primary conjugate body does not contain the connector, the third antibody binds to the first antibody of the primary conjugate body to amplify a signal.

In a signal amplification method of the present invention, a primary conjugate body containing a first antibody and a first indicator binds to an antigen in an immunochromatograph and then binds to a second antibody (i.e., an immobilized capture antibody) in a sandwich reaction and then a secondary conjugate body containing a third antibody and a second indicator binds to the primary conjugate body to amplify a signal. In particular, the signal amplification method of the present invention increases the sensitivity of the signal amplification by controlling the flow rate of the secondary conjugate body by controlling the indicator size of the secondary conjugate body to be larger than the indicator size of the primary conjugate body.

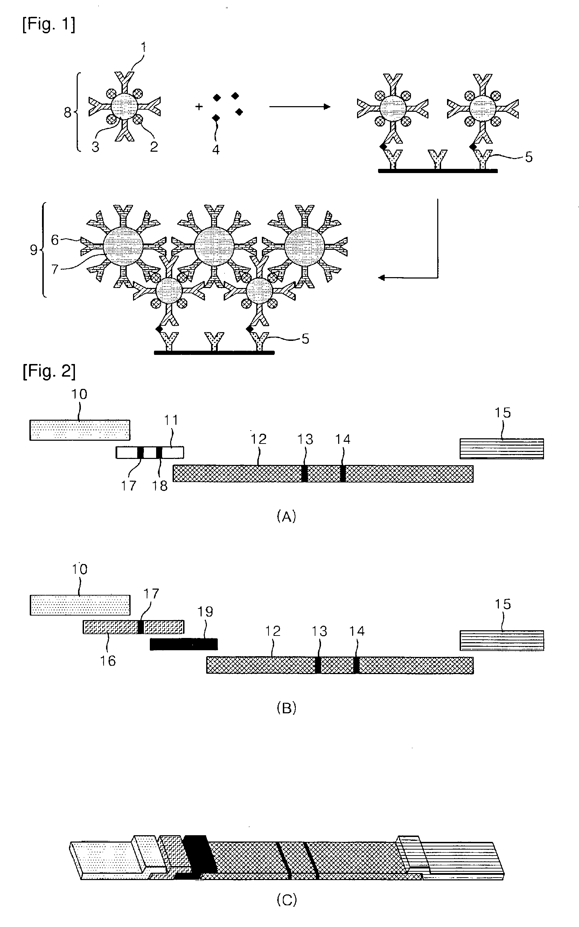

FIG. 1 is a diagram illustrating a process of signal amplification in assaying an antigen by a method of the present invention including a connector. In FIG. 1, a first antibody 1 and a connector 2 bind to a first indicator 3 to form a primary conjugate body 8, and a third antibody 6 binds to a second indicator 7 to form a secondary conjugate body 9. The primary conjugate body 8 binds to an analyte 4 (i.e., an antigen) and binds to a second antibody 5 (i.e., an immobilized capture antibody), and then the secondary conjugate body 9 binds to the primary conjugate body 8.

A process of an immunochromatographic signal amplification method according to the present invention will be described below in detail with reference to the accompanying drawings. The primary conjugate body 8, which has the first antibody 1 capable of binding specifically to a first epitope of the analyte 4, the connector 2, and the first indicator 3 to which the first antibody 1 and the connector 2 are bound, binds to the analyte 4; while flowing through a strip, the analyte 4 bound to the primary conjugate body 8 binds to the immobilized second antibody 5 binding specifically to a second epitope of the analyte 4; and then the secondary conjugate body 9, which has the third antibody 6 binding specifically to the connector 2 of the primary conjugate body 8, and the second indicator 7 to which the third antibody 6 is bound, binds to the connector 2 of the primary conjugate body 8. Herein, the primary conjugate body 8 is disposed nearer to the immobilized second antibody 5 than the secondary conjugate body 9, and the particle of the second indicator 7 is larger than the particle of the first indicator 3. Thus, the secondary conjugate body 9 reaches the immobilized second antibody 5 later than the primary conjugate body 8, thereby amplifying an immunochromatographic signal.

The connector 2 included in the primary conjugate body 8 may be a bovine serum albumin (BSA) or a human serum albumin (HAS), and the third antibody 6 may be an anti-BSA or an anti-HSA. However, the connector 2 and the third antibody 6 are not limited thereto. For example, the connector 2 and the third antibody 6 may comprise any substances which react selectively each other such as a peptide-antibody binding and a biotin-avidin binding that enables the secondary conjugate body 9 to bind to the primary conjugate body 8, while not to bind to the analyte 4. For example, according to the present invention, a serum albumin (specifically a BSA) may be used as the connector 2 and the secondary conjugate body 9 may be produced using another serum albumin that is not used to produce the primary conjugate body 8. However, because the connector 2 is included in the primary conjugate body 8 to enable the secondary conjugate body 9 to bind to the primary conjugate body 8, the connector 2 may not be included if the third antibody 6 and the first antibody 1 can bind specifically to each other. The case where the third antibody 6 and the first antibody 1 can bind specifically to each other includes the case where the third antibody 6 recognizes the first antibody 1 as an antigen thus bind to the first antibody 1. As described above, the present invention may include any structure that enables the secondary conjugate body 9 to bind to the primary conjugate body 8.

The analyte 4 capable of being detected by the method of the present invention may be any analyte that can bind to the first antibody 1 and the second antibody 5 by an immunoreaction, i.e., an antigen-antibody reaction to form a sandwich-type immune complex. Examples of the analyte 4 include proteins, deoxyribo nucleic acids (DNAs), environmental pollutants including environmental hormones, disease factors such as viruses and food poisoning bacteria, and pathogenic poisonous substances.

In the present invention, the antibody and the antigen may be any substances that can bind specifically to the analyte 4 by an antigen-antibody reaction. If the analyte 4 is an antibody, a substance binding specifically to the antibody may be used as an antigen. According to the analyte 4, the antigen and antibody may be any well-known antigen and antibody.

In the present invention, examples of a coloring substance generating an 'indicator' signal include a color former, a fluor, and a colored latex particle. For example, the coloring substance may be a gold colloid or a quantum dot. The first indicator 3 of the primary conjugate body 8 and the second indicator 7 of the secondary conjugate body 9 may be different from each other. However, in consideration of the problems in fabrication, the first indicator 3 and the second indicator 7 may be identical to each other.

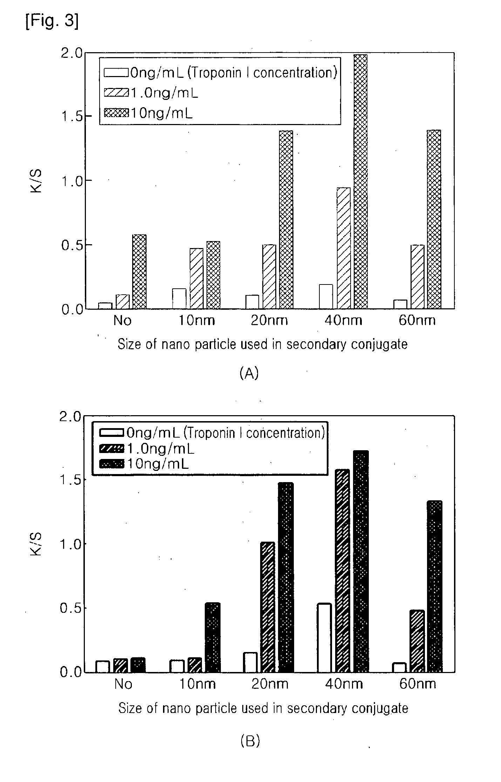

As can be seen from an embodiment 3 and FIG. 3, the particle of the first indicator 3 may have a size of about 10 nm to about 20 nm and the particle of the second indicator 7 may have a size of about 20 nm to about 60 nm. In the present invention, the size of the particle of the second indicator 7 is larger than the size of the particle of the first indicator 3, so that the secondary conjugate body 9 develops on the strip slower than the primary conjugate body 8. Thus, the primary conjugate body 8 reaches the second antibody 5 together with the antigen and binds to the second antibody 5, and then the secondary conjugate body 9 binds to the primary conjugate body 8.

An immunochromatographic kit of the present invention uses the above signal amplification method. The immunochromatographic kit of the present invention includes: a a sample pad to which a liquid sample containing an analyte is applied; a conjugate pad including a primary conjugate body having a first antibody binding specifically to a first epitope of an analyte, a connector, and a first indicator to which the first antibody and the connector are bound, and a secondary conjugate body having a third antibody binding specifically to the connector of the primary conjugate body and a second indicator to which the third antibody is bound, wherein the primary conjugate body is disposed nearer to an immobilized second antibody than the secondary conjugate body and the second indicator is larger than the first indicator so that the secondary conjugate body reaches the immobilized second antibody later than the primary conjugate body; a membrane including a detection site immobilizing thereto the second antibody binding specifically to a second epitope of the analyte to which the primary conjugate body is bound, and a control site for error detection; and an absorbing pad absorbing the liquid sample by a capillary phenomenon.

FIG. 2A is a diagram illustrating a structure of an immunochromatographic kit according to an embodiment of the present invention, in which a reference numeral 10 denotes a sample pad, a reference numeral 11 denotes a conjugate pad, a reference numeral 12 denotes a membrane, a reference numeral 13 denotes a detection site, a reference numeral 14 denotes a control site, a reference numeral 15 denotes an absorbing pad, a reference numeral 17 denotes a site immobilizing a secondary conjugate body, and a reference numeral 18 denotes a site immobilizing a primary conjugate body. FIG. 2B is a diagram illustrating a structure of an immunochromatographic kit having two conjugate pads according to another embodiment of the present invention, in which a reference numeral 16 denotes a second conjugate pad, a reference numeral 17 denotes a site immobilizing a secondary conjugate body, and a reference numeral 19 denotes a first conjugate pad applied entirely with a primary conjugate body. FIG. 2C is a diagram illustrating an assembly structure of the immunochromatographic kit of FIG. 2B.

The pad used to fabricate the immunochromatographic kit of the present invention may be formed of any natural porous material or any synthetic porous material. For example, the pad may be formed of a nitrocellulose.

The sample pad 10 is a portion that first absorbs a liquid sample containing an analyte. One end of the membrane 12 may be used as the sample pad 10, or a separate member may be used as the sample pad 10. The sample pad 10 absorbs a sample containing an analyte, and moves the analyte to the conjugate pad 11 by a capillary phenomenon.

The conjugate pad 11 has a secondary conjugate body and a primary conjugate body that are in a dry state. When the conjugate pad 11 absorbs a liquid sample containing an analyte, the secondary conjugate body and the primary conjugate body become fluid and move to the membrane 12. The primary conjugate body is disposed nearer to an immobilized second antibody than the secondary conjugate body, and the particle of the second indicator is produced to be larger than the particle of the first indicator, so that the secondary conjugate body develops on the strip slower than the primary conjugate body.

As can be seen from the embodiment 3 and FIG. 3, the particle of the second indicator 7 may have a size of about 20 nm to about 60 nm and the particle of the first indicator 3 may have a size of about 10 mn to about 20 nm. Thus, by controlling the arrangement of the conjugate bodies and the particle sizes of the indicators included in the conjugate bodies, the primary conjugate body and the secondary conjugate body do not contact each other, and the primary conjugate body bound to the antigen reaches the capture antigen and then the secondary conjugate body binds thereto.

As illustrated in FIG. 2A, the primary conjugate body and the secondary conjugate body may be disposed at separate regions of a single conjugate pad. Alternatively, as illustrated in FIG. 2B, the primary conjugate body is included in the first conjugate pad and the secondary conjugate body is included in the second conjugate pad that is separate from the first conjugate pad. The assembly of the strip of FIG. 2B results in the structure of FIG. 2C. It is preferable that the primary conjugate body and the secondary conjugate body move sequentially with a time interval. Therefore, it is preferable that the conjugate pad is fabricated such that the primary conjugate body and the secondary conjugate body do not contact each other during the movement of the liquid sample on the strip and thus the primary conjugate body and the secondary conjugate body do not mix with each other.

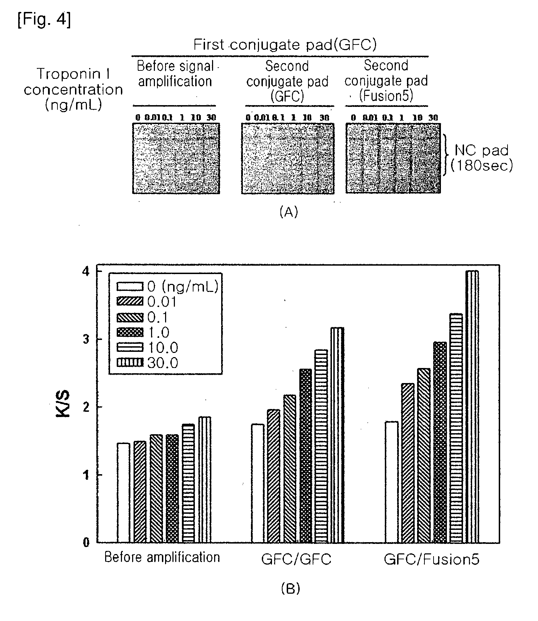

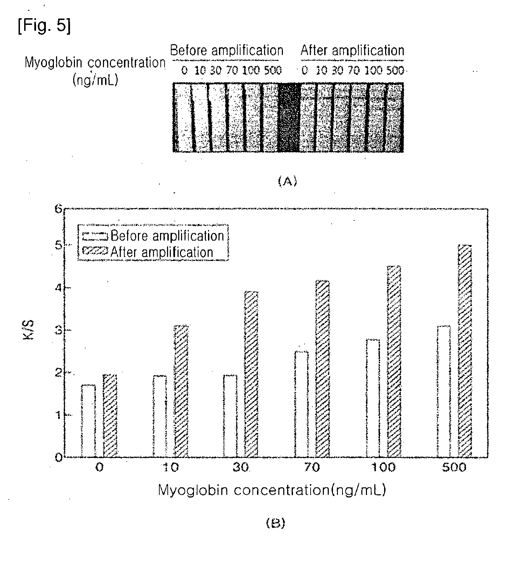

It can be seen from an embodiment 4 that the signal amplification effect is enhanced when the contact between a primary conjugate body and a secondary conjugate body are further controlled by separating the primary conjugate body and the secondary conjugate body by means of two separate conjugate pads. In result, the signal amplification effect is good when the first conjugate pad and the secondary conjugate body are separated by means of two conjugate pads (see FIGS. 4 and 5). The first conjugate pad may be a pad that has a pore size of about 20 µm or more, which is suitable to process a substance such as a polyvinyl alcohol (PVA) without retarding the flow of a solvent and an antigen, and smoothly maintains the development of a gold nano-particle conjugate body. For example, the first conjugate pad may be a glass fiber conjugate (GFC) pad. The second conjugate pad may be a pad that has an effect of filtering a bead particle and a bio particle of about 2 µm or more and thus has a function of separating and filtering a plasma when sampling a blood; and has a good liquid absorbing power and thus is suitable to develop a gold nano-particle conjugate body. For example, the second conjugate pad may be a Fusion 5 pad. The conjugate pads are not limited thereto but may be any other suitable conjugate pad that has the above properties.

The membrane 12 may be formed of a substance coated with nitrocellulose particles. The membrane 12 may be formed of any substance that can maintain a sufficient reaction time between an antigen and an antibody at a lateral flow rate of about 180 sec/cm and thus can secure the fluidity of a liquid sample. The membrane 12 includes: a detection site immobilizing thereto a second antibody bonding specifically to a second region of an analyte to which a primary conjugate body is bound; and a control site detecting the presence of a reaction due to an error as a contrast group thereof. The detection site shows the result for determination of a test result. The control site is configured to detect an error of a capture antibody and a gold nano-particle conjugate body, and is used to detect whether mobile substances react to the detection site/the control site without an error.

The absorbing pad 15 may be any pad that can sufficiently absorb post-reaction residuals.

Hereinafter, the present invention will be described in more detail with reference to embodiments. These embodiments are merely exemplary, and the present invention is not limited by the embodiments.

### Mode for the Invention

**Embodiment 1: Composition of Nano Particle-Antibody Conjugate Body**

1. Composition of Primary Conjugate Body

A 0.1 mL 0.1 M borate buffer (pH 8.5) was added into a 1 mL gold nano-particle colloid solution (BBInternational, 10 nm), a 1 mg/mL first antibody 10 uL was added thereinto, and they were reacted for 30 minutes. After the reaction, a 0.1 ml solution obtained by dissolving a 1%(w/v) bovine serum albumin (BSA) (Sigma) as a connector in a phosphate buffered saline (PBS) (Gibco) was added thereinto, and they were reacted at the normal temperatures for 15 minutes. After the reaction, it was centrifuged at 10,000 rpm at 4°C for 20 minutes to disperse in a 1 mL BSA (Sigma) solution dissolved in a 10 mM PBS at a 1 mg/mL concentration. The centrifuging/ dispersing process was repeated once again, and it was again centrifuged to disperse in a 1 mL PBS, thereby fabricating a primary conjugate body.

The first antibody may be 4T21, 560 (HyTest) for immunoassay of a troponin I, and may be M012607 (Fitzgerald) for immunoassay of a myoglobin.

2. Composition of Secondary Conjugate Body

A 0.1 mL 0.1 M borate buffer (pH 8.5) was added into a 1 mL gold nano-particle colloid solution (BBInternational, 10, 20, 40, 60 nm), an anti-BSA antibody (Genetex) 10 uL as a 1 mg/mL third antibody was added thereinto, and they were reacted for 30 minutes. After the reaction, a 0.1 ml solution obtained by dissolving a 1%(w/v) human serum albumin (HSA) (Sigma) in a distilled water was added thereinto, and they were reacted at the normal temperatures for 15 minutes. After the reaction, it was centrifuged at 10,000 rpm at 4°C for 20 minutes to disperse in a 1 mL solution dissolved in a PBS at a 1 mg/mL HSA concentration. The centrifuging/dispersing process was repeated twice, thereby fabricating a secondary conjugate body.

**Embodiment 2: Fabrication of Immunochromatograph**

1. Method of Fabricating Immunochromatograph

A nitrocellulose membrane (Millipore, 180 sec) and an absorbing pad (Millipore) were adhered to a plastic pad (Millipore). Thereafter, using a dispenser system (Zeta Co.), a capture antibody (second antibody) 1 mg/mL solution dissolved in a PBS and a goat anti-mouse IgG antibody (Sigma, M8642) 1 mg/mL solution dissolved in a PBS as a contrast group were scribed on the membrane at a speed of 6 cm/sec, thereby forming a detection site and a control line. After the membrane was dried, it was cut by a cutter at intervals of 3 mm.

The second antibody, i.e., the capture antibody, may be a troponin capture antibody (Hytest) for immunoassay of a troponin I, and may be a myoglobin capture antibody M09983110 (Fitzgerald) for immunoassay of a myoglobin.

After a sample pad (Millipore, C068) was immersed in a 0.5% Tween 20, a 5% Sucrose, a 0.05% Dextran, a 5% sodium azide aqueous solution, it was dried and cut to about 10×3 mm.

As illustrated in FIG. 2B, the conjugated pad and the sample pad were assembled with the plastic pad, i.e., an assembly of the membrane and the absorbing pad. The difference from FIG. 2B is a second conjugate pad fabrication method. The second conjugate pad fabrication method will be described below in more detail.

2. Method of Fabricating Conjugate Pad

Conjugate pads were fabricated by discrimination between a first conjugate pad and a second conjugate pad. For the primary and second conjugate pads, a GFC (Millipore Co.) was cut to 5x3 mm, and they were dried after being coated with the primary conjugate body and the secondary conjugate body of the embodiment 1 by 5 µL.

**Embodiment 3: Signal Amplification Effect according to the size of gold nano-particle**

A primary conjugate body using gold nano-particles with a diameter of 10 nm or 20 nm, and gold nano-particles forming a secondary conjugate body were fabricated in various sizes (10, 20, 40, 60 nm), and the signal amplification effects depending on the gold nano-particle sizes were observed.

The immunochromatographic kit was immersed in a 96 well plate where a serum (Linear chemicals, Cromatest) 70 µL dissolving a troponin I at a predetermined concentration was immersed, and a measurement was performed. The result of the case of the primary conjugate body having a nano-particle diameter of 10 mn is illustrated in Table 1 and FIG. 3A that illustrates the K/S values depending on the nano-particle sizes (the primary conjugate body has a nano-particle diameter of 10 nm). The result of the case of the primary conjugate body having a nano-particle diameter of 20 nm is illustrated in Table 2 and FIG. 3B that illustrates the K/S values depending on the nano-particle sizes (the primary conjugate body has a nano-particle diameter of 20 nm).

**Table 1**

| Concentration of Troponin I\Size (Diameter) of Nano Particle | 10 nm | 20 nm | 40 nm | 60 nm |
|---|---|---|---|---|
| 0 ng/mL | 0.1554 | 0.1101 | 0.1918 | 0.0694 |
| 1.0 ng/mL | 0.4745 | 0.5004 | 0.9431 | 0.5004 |
| 10 ng/mL | 0.5248 | 1.3889 | 1.9793 | 1.3889 |

K/S=(1-Rd)²/2Rd; Rd: Relative Diffusion Reflectance, K: Absorption Coefficient of Sample, S: Scattering Coefficient

**Table 2**

| Concentration of Troponin I\size (Diameter) of Nano Particle | 10 nm | 20 nm | 40 nm | 60 nm |
|---|---|---|---|---|
| 0 ng/mL | 0.0897 | 0.1501 | 0.5311 | 0.0663 |
| 1.0 ng/mL | 0.1092 | 1.0054 | 1.5717 | 0.4782 |
| 10 ng/mL | 0.5338 | 1.4725 | 1.7231 | 1.3273 |

As a result of detection of the measurement result after 10 minutes on the basis of the K/S values (=(1-Rd)²/2Rd; Rd: Relative Diffusion Reflectance, K: Absorption Coefficient of Sample, S: Scattering Coefficient), the sensitivity of a combination of a primary conjugate body with a 10 nm gold nano-particle size and a secondary conjugate body with a 40 nm gold nano-particle size increased highest in comparison with those of the other combinations, and the sensitivity increased two or more times of a log value (about 100 times) in comparison with the case of not amplifying a signal.

**Embodiment 4: Signal Amplification Effect according to the separation of Conjugate Bodies**

(1) Fabrication of Conjugate Pad

A conjugate pad was fabricated in the following way in order to observe the signal amplification effect of the case where a conjugate pad is fabricated such that a primary conjugate body and a secondary conjugate body do not contact each other. An immunochromatograph not enhancing was fabricated as a contrast group. Herein, a conjugate pad was cut to 5x3 mm by means of a GFC pad (Millipore), and then it was dried after being coated with a primary conjugate body fabricated using an antibody corresponding to the analyte in the embodiment 1.

1) For fabrication of a conjugate pad where a primary conjugate body and a secondary conjugate body do contact, two conjugate pads (GFC, Millipore GFC203000) were cut to 5×3 mm, and then they were dried after being respectively coated the primary conjugate body and the secondary conjugate body fabricated according to the embodiment 1. If the conjugate pads are entirely coated, there is a possibility that the overlaps between the two conjugate pads may contact each other so that the primary conjugate body and the secondary conjugate body are mixed during the inflow of liquid sample containing an analyte.

2) For fabrication of a conjugate pad where a primary conjugate body and a secondary conjugate body do not contact, using the primary conjugate body and the secondary conjugate body fabricated according to the embodiment 1, the primary conjugate body was coated to 5 µL according to the method of the embodiment 2 and were dried to fabricate a first conjugate pad. Using a Fusion 5™ pad (Whatman Co.) as a second conjugate pad, the secondary conjugate body was formed in a line to fabricate an immunochromatograph, and an experiment was performed. The structure of the immunochromatographic kit using the conjugate pads is identical to that of FIGS. 2B and 2C. The nano-particle size of the primary conjugate body was 10 nm, and the nano-particle size of the primary conjugate body was 40 nm. If the immunochromatographic kit is fabricated as described above, the secondary conjugate body is formed in a line on the second conjugate pad, so that it can be prevented from contacting the first conjugate pad where the primary conjugate body is immersed.

(2) Troponin I Assay

The so-fabricated immunochromatograph and the contrast group immunochromatograph were immersed in a 96 well plate where a plasma (Linear chemicals, Cromatest) 70 µL dissolving a troponin I antigen at a predetermined concentration was immersed, and a measurement was performed.

As a result of detection of the measurement result after 10 mimutes, as can be seen from FIG. 4A, the sensitivity of the case of amplifying a signal increases in comparison with the contrast group, the signal sensitivity of the case of using the Fusion 5™ pad as the second conjugate pad so that the primary conjugate body and the secondary conjugate body do not contact each other is most desirable. FIG. 4B is a graph illustrating the above result.

(3) Myoglobin Assay

In the above method, the myoglobin of the case of not amplifying a signal and the myoglobin of the case of using the Fusion 5™ pad as the second conjugate pad to amplify a signal were assayed. The first antibody of the primary conjugate body and the second antibody, i.e., the capture antibody were anti-myoglobin antigens of Fitzgerald product.

As a result of detection of the measurement result after 10 minutes, as can be seen from FIG. 5A, a signal is amplified in comparison with the contrast group not amplifying a signal. FIG. 5B is a graph illustrating the above result.

While the present invention has been shown and described in connection with the exemplary embodiments, it will be apparent to those skilled in the art that modifications and variations can be made without departing from the spirit and scope of the invention as defined by the appended claims.

## Claims

1. A method for amplifying a signal in an immunochromatographic assay, comprising:
binding a primary conjugate body, which has a first antibody binding specifically to a first epitope of an analyte, a connector, and a first indicator particle to which the first antibody and the connector are bound, to the analyte;
binding the analyte bound to the primary conjugate body to an immobilized second antibody binding specifically to a second epitope of the analyte; and
binding a secondary conjugate body, which has a third antibody binding specifically to the connector of the primary conjugate body and a second indicator particle to which the third antibody is bound, to the connector of the primary conjugate body,
wherein the primary conjugate body is disposed nearer to the immobilized second antibody than the secondary conjugate body, and the particle of the first indicator has a size of about 10 nm to about 20 nm and the particle of the second indicator has a size of about 20 nm to about 60 nm wherein the particle of the second indicator is larger than the particle of the first indicator, so that the secondary conjugate body reaches the immobilized second antibody later than the primary conjugate body.

2. A method for amplifying a signal in an immunochromatographic assay, comprising:
binding a primary conjugate body, which has a first antibody binding specifically to a first epitope of an analyte and a first indicator particle to which the first antibody is bound, to the analyte;
binding the analyte bound to the primary conjugate body to an immobilized second antibody binding specifically to a second epitope of the analyte; and
binding a secondary conjugate body, which has a third antibody binding specifically to the first antibody of the primary conjugate body and a second indicator particle to which the third antibody is bound, to the first antibody of the primary conjugate body,
wherein the primary conjugate body is disposed nearer to the immobilized second antibody than the secondary conjugate body, and the particle of the first indicator has a size of about 10 nm to about 20 nm and the particle of the second indicator has a size of about 20 nm to about 60 nm wherein the particle of the second indicator is larger than the particle of the first indicator, so that the secondary conjugate body reaches the immobilized second antibody later than the primary conjugate body.

3. The method of claim 1, wherein the connector is selected from the group consisting of a bovine serum albumin (BSA), a human serum albumin (HSA), a biotin, an avidin and a peptide, and does not bind to the analyte.

4. The method of claim 1 or 2, wherein the indicators are gold colloids or a quantum dots.

5. The method of claim 1 or 2, wherein the analyte is selected from the group consisting of an antigen protein, a deoxyribo nucleic acid (DNA), an environmental hormone, a pathogenic poisonous substance and a food poisoning bacterium.

6. An immunochromatographic kit comprising:
a sample pad to which a liquid sample containing an analyte is applied;
a conjugate pad comprising a primary conjugate body having a first antibody binding specifically to a first epitope of an analyte, a connector, and a first indicator to which the first antibody and the connector are bound, and a secondary conjugate body having a third antibody binding specifically to the connector of the primary conjugate body and a second indicator to which the third antibody is bound, wherein the primary conjugate body is disposed nearer to an immobilized second antibody than the secondary conjugate body and the particle of the first indicator has a size of about 10 nm to about 20 nm and the particle of the second indicator has a size of about 20 nm to about 60 nm wherein the second indicator is larger than the first indicator so that the secondary conjugate body reaches the immobilized second antibody later than the primary conjugate body;
a membrane comprising a detection site immobilizing thereto the second antibody binding specifically to a second epitope of the analyte to which the primary conjugate body is bound, and a control site for error detection; and
an absorbing pad absorbing the liquid sample by a capillary phenomenon.

7. An immunochromatographic kit comprising:
a sample pad to which a liquid sample containing an analyte is applied;
a conjugate pad comprising a primary conjugate body having a first antibody binding specifically to a first epitope of an analyte and a first indicator to which the first antibody is bound, and a secondary conjugate body having a third antibody binding specifically to the first antibody of the primary conjugate body and a second indicator to which the third antibody is bound, wherein the primary conjugate body is disposed nearer to an immobilized second antibody than the secondary conjugate body and the particle of the first indicator has a size of about 10 nm to about 20 nm and the particle of the second indicator has a size of about 20 nm to about 60 nm wherein the second indicator is larger than the first indicator so that the secondary conjugate body reaches the immobilized second antibody later than the primary conjugate body;
a membrane comprising a detection site immobilizing thereto the second antibody binding specifically to a second epitope of the analyte to which the primary conjugate body is bound, and a control site for error detection; and
an absorbing pad absorbing the liquid sample by a capillary phenomenon.

8. The immunochromatographic kit of claim 6, wherein the connector is a bovine serum albumin (BSA).

9. The immunochromatographic kit of claim 7 or 8, wherein the conjugate pad comprises a first conjugate pad including the primary conjugate body and a second conjugate pad including the secondary conjugate body, and is fabricated such that the primary conjugate body and the secondary conjugate body do not contact each other.

10. The immunochromatographic kit of claim 7 or 8, wherein the indicators are gold colloids or a quantum dots.

11. The immunochromatographic kit of claim 7 or 8, wherein the analyte is selected from the group consisting of an antigen protein, a deoxyribo nucleic acid (DNA), an environmental hormone, a pathogenic poisonous substance and a food poisoning bacterium.

## Patentansprüche

1. Verfahren zur Verstärkung eines Signals in einem immunchromatographischen Assay, umfassend:
Binden eines primären Konjugatkörpers, der einen ersten Antikörper, welcher spezifisch an ein erstes Epitop eines Analyten bindet, einen Konnektor und einen ersten Indikatorpartikel, an den der erste Antikörper und der Konnektor gebunden sind, aufweist, an den Analyten;
Binden des Analyten, der an den primären Konjugatkörper gebunden ist, an einen immobilisierten zweiten Antikörper, der spezifisch an ein zweites Epitop eines Analyten bindet; und
Binden eines sekundären Konjugatkörpers, der einen dritten Antikörper, welcher spezifisch an den Konnektor des primären Konjugatkörpers bindet, und einen zweiten Indikatorpartikel, an den der dritte Antikörper gebunden ist, aufweist, an den Konnektor des primären Konjugatkörpers;
wobei sich der primäre Konjugatkörper näher bei dem immobilisierten zweiten Antikörper befindet als der sekundäre Konjugatkörper und der Partikel des ersten Indikators eine Größe von etwa 10 nm bis etwa 20 nm aufweist und der Partikel des zweiten Indikators eine Größe von etwa 20 nm bis etwa 60 nm aufweist, wobei der Partikel des zweiten Indikators größer ist als der Partikel des ersten Indikators, so dass der sekundäre Konjugatkörper den immobilisierten zweiten Antikörper später erreicht als der primäre Konjugatkörper.

2. Verfahren zur Verstärkung eines Signals in einem immunchromatographischen Assay, umfassend:
Binden eines primären Konjugatkörpers, der einen ersten Antikörper, welcher spezifisch an ein erstes Epitop eines Analyten bindet, und einen ersten Indikatorpartikel, an den der erste Antikörper gebunden ist, aufweist, an den Analyten;
Binden des Analyten, der an den primären Konjugatkörper gebunden ist, an einen immobilisierten zweiten Antikörper, der spezifisch an ein zweites Epitop eines Analyten bindet; und
Binden eines sekundären Konjugatkörpers, der einen dritten Antikörper, welcher spezifisch an den ersten Antikörper des primären Konjugatkörpers bindet, und einen zweiten Indikatorpartikel, an den der dritte Antikörper gebunden ist, aufweist, an den ersten Antikörper des primären Konjugatkörpers;
wobei sich der primäre Konjugatkörper näher bei dem immobilisierten zweiten Antikörper befindet als der sekundäre Konjugatkörper und der Partikel des ersten Indikators eine Größe von etwa 10 nm bis etwa 20 nm aufweist und der Partikel des zweiten Indikators eine Größe von etwa 20 nm bis etwa 60 nm aufweist, wobei der Partikel des zweiten Indikators größer ist als der Partikel des ersten Indikators, so dass der sekundäre Konjugatkörper den immobilisierten zweiten Antikörper später erreicht als der primäre Konjugatkörper.

3. Verfahren gemäß Anspruch 1, wobei der Konnektor aus der Gruppe, die aus einem Rinderserumalbumin (BSA), einem Humanserumalbumin (HSA), einem Biotin, einem Avidin und einem Peptid besteht, ausgewählt ist und nicht an den Analyten bindet.

4. Verfahren gemäß Anspruch 1 oder 2, wobei es sich bei den Indikatoren um Goldkolloide oder Quantenpunkte handelt.

5. Verfahren gemäß Anspruch 1 oder 2, wobei der Analyt aus der Gruppe ausgewählt ist, die aus einem Antigenprotein, einer Desoxyribonucleinsäure (DNA), einem Umwelthormon, einer pathogenen giftigen Substanz und einem lebensmittelvergiftenden Bakterium besteht.

6. Immunchromatographischer Kit, umfassend:
ein Probenfeld, auf das eine flüssige Probe, die einen Analyten enthält, aufgetragen wird;
ein Konjugatfeld, das einen primären Konjugatkörper, der einen ersten Antikörper, welcher spezifisch an ein erstes Epitop eines Analyten bindet, einen Konnektor und einen ersten Indikator, an den der erste Antikörper und der Konnektor gebunden sind, aufweist, und einen sekundären Konjugatkörper, der einen dritten Antikörper, welcher spezifisch an den Konnektor des primären Konjugatkörpers bindet, und einen zweiten Indikator, an den der dritte Antikörper gebunden ist, aufweist, umfasst, wobei sich der primäre Konjugatkörper näher bei dem immobilisierten zweiten Antikörper befindet als der sekundäre Konjugatkörper und der Partikel des ersten Indikators eine Größe von etwa 10 nm bis etwa 20 nm aufweist und der Partikel des zweiten Indikators eine Größe von etwa 20 nm bis etwa 60 nm aufweist, wobei der Partikel des zweiten Indikators größer ist als der Partikel des ersten Indikators, so dass der sekundäre Konjugatkörper den immobilisierten zweiten Antikörper später erreicht als der primäre Konjugatkörper;
eine Membran, die eine Nachweisstelle, an der der zweite Antikörper, der spezifisch an ein zweites Epitop des Analyten, an den der primäre Konjugatkörper gebunden ist, bindet, immobilisiert ist, und eine Kontrollstelle zum Fehlernachweis umfasst; und
ein Aufsaugfeld, das die flüssige Probe durch ein Kapillarphänomen aufsaugt.

7. Immunchromatographischer Kit, umfassend:
ein Probenfeld, auf das eine flüssige Probe, die einen Analyten enthält, aufgetragen wird;
ein Konjugatfeld, das einen primären Konjugatkörper, der einen ersten Antikörper, welcher spezifisch an ein erstes Epitop eines Analyten bindet, und einen ersten Indikator, an den der erste Antikörper gebunden ist, aufweist, und einen sekundären Konjugatkörper, der einen dritten Antikörper, welcher spezifisch an den ersten Antikörper des primären Konjugatkörpers bindet, und einen zweiten Indikator, an den der dritte Antikörper gebunden ist, aufweist, umfasst, wobei sich der primäre Konjugatkörper näher bei einem immobilisierten zweiten Antikörper befindet als der sekundäre Konjugatkörper und der Partikel des ersten Indikators eine Größe von etwa 10 nm bis etwa 20 nm aufweist und der Partikel des zweiten Indikators eine Größe von etwa 20 nm bis etwa 60 nm aufweist, wobei der Partikel des zweiten Indikators größer ist als der Partikel des ersten Indikators, so dass der sekundäre Konjugatkörper den immobilisierten zweiten Antikörper später erreicht als der primäre Konjugatkörper;
eine Membran, die eine Nachweisstelle, an der der zweite Antikörper, der spezifisch an ein zweites Epitop des Analyten, an den der primäre Konjugatkörper gebunden ist, bindet, immobilisiert ist, und eine Kontrollstelle zum Fehlernachweis umfasst; und
ein Aufsaugfeld, das die flüssige Probe durch ein Kapillarphänomen aufsaugt.

8. Immunchromatographischer Kit gemäß Anspruch 6, wobei der Konnektor ein Rinderserumalbumin (BSA) ist.

9. Immunchromatographischer Kit gemäß Anspruch 7 oder 8, wobei das Konjugatfeld ein erstes Konjugatfeld, das den primären Konjugatkörper umfasst, und ein zweites Konjugatfeld, das den sekundären Konjugatkörper umfasst, umfasst und so hergestellt ist, dass der primäre Konjugatkörper und der sekundäre Konjugatkörper einander nicht berühren.

10. Immunchromatographischer Kit gemäß Anspruch 7 oder 8, wobei es sich bei den Indikatoren um Goldkolloide oder Quantenpunkte handelt.

11. Immunchromatographischer Kit gemäß Anspruch 7 oder 8, wobei der Analyt aus der Gruppe ausgewählt ist, die aus einem Antigenprotein, einer Desoxyribonucleinsäure (DNA), einem Umwelthormon, einer pathogenen giftigen Substanz und einem lebensmittelvergiftenden Bakterium besteht.

## Revendications

1. Procédé d'amplification d'un signal dans un essai immunochromatographique, comprenant :
la liaison, à la substance à analyser, d'un corps conjugué primaire, qui a une première liaison d'anticorps spécifiquement avec un premier épitope d'une substance à analyser, d'un connecteur et d'une première particule indicatrice auxquels sont liés le premier anticorps et le connecteur ;
la liaison de la substance à analyser liée au corps conjugué primaire à un deuxième anticorps immobilisé se liant spécifiquement à un deuxième épitope de la substance à analyser ; et
la liaison d'un corps conjugué secondaire, qui a une troisième liaison d'anticorps spécifiquement avec le connecteur du corps conjugué primaire et d'une deuxième particule indicatrice à laquelle est lié le troisième anticorps, au connecteur du corps conjugué primaire,
dans lequel le corps conjugué primaire est disposé plus près du deuxième anticorps immobilisé que le corps conjugué secondaire, et dans lequel la particule du premier indicateur a une taille d'environ 10 nm à environ 20 nm et la particule du deuxième indicateur a une taille d'environ 20 nm à environ 60 nm, dans lequel la particule du deuxième indicateur est plus grande que la particule du premier indicateur, de telle sorte que le deuxième corps conjugué atteigne le deuxième anticorps immobilisé après le corps conjugué primaire.

2. Procédé d'amplification d'un signal dans un essai immunochromatographique, comprenant :
la liaison, à la substance à analyser, d'un corps conjugué primaire, qui a une première liaison d'anticorps spécifiquement avec un premier épitope d'une substance à analyser et d'une première particule indicatrice à laquelle est lié le premier anticorps ;
la liaison de la substance à analyser liée au corps conjugué primaire à un deuxième anticorps immobilisé se liant spécifiquement à un deuxième épitope de la substance à analyser ; et
la liaison d'un corps conjugué secondaire, qui a une troisième liaison d'anticorps spécifiquement avec le premier anticorps du corps conjugué primaire et d'une deuxième particule indicatrice à laquelle est lié le troisième anticorps, au premier anticorps du corps conjugué primaire,
dans lequel le corps conjugué primaire est disposé plus près du deuxième anticorps immobilisé que le corps conjugué secondaire, et dans lequel la particule du premier indicateur a une taille d'environ 10 nm à environ 20 nm et la particule du deuxième indicateur a une taille d'environ 20 nm à environ 60 nm, dans lequel la particule du deuxième indicateur est plus grande que la particule du premier indicateur, de telle sorte que le deuxième corps conjugué atteigne le deuxième anticorps immobilisé après le corps conjugué primaire.

3. Procédé selon la revendication 1, dans lequel le connecteur est choisi dans le groupe constitué d'une albumine de sérum bovin (BSA), d'une albumine de sérum humain (HSA), d'une biotine, d'une avidine et d'un peptide, et ne se lie pas au produit à analyser.

4. Procédé selon les revendications 1 ou 2, dans lequel les indicateurs sont des colloïdes d'or ou des points quantiques.

5. Procédé selon les revendications 1 ou 2, dans lequel la substance à analyser est choisie dans le groupe constitué d'une protéine antigène, d'un acide désoxyribonucléique (ADN), d'une hormone environnementale, d'une substance poison et d'une bactérie empoisonnant des aliments.

6. Kit immunochromatographique comprenant :
un tampon pour échantillon sur lequel est appliqué un échantillon de liquide contenant la substance à analyser ;
un tampon conjugué comprenant un corps conjugué primaire ayant une première liaison d'anticorps spécifiquement avec un premier épitope d'une substance à analyser, un connecteur et un premier indicateur auxquels sont liés le premier anticorps et le connecteur, et un corps conjugué secondaire ayant une troisième liaison d'anticorps spécifiquement avec le connecteur du corps conjugué primaire et un deuxième indicateur auquel est lié le troisième anticorps, dans lequel le corps conjugué primaire est disposé plus près d'un deuxième anticorps immobilisé que le corps conjugué secondaire, et dans lequel la particule du premier indicateur a une taille d'environ 10 nm à environ 20 nm et la particule du deuxième indicateur a une taille d'environ 20 nm à environ 60 nm, dans lequel le deuxième indicateur est plus grand que le premier indicateur, de telle sorte que le deuxième corps conjugué atteigne le deuxième anticorps immobilisé après le corps conjugué primaire ;
une membrane comprenant un site de détection immobilisant en cet endroit le deuxième anticorps se liant spécifiquement à un deuxième épitope de la substance à analyser auquel est lié le corps conjugué primaire, et un site de contrôle pour la détection d'erreur ; et
un tampon absorbant qui absorbe l'échantillon de liquide par un phénomène capillaire.

7. Kit immunochromatographique comprenant :
un tampon pour échantillon sur lequel est appliqué un échantillon de liquide contenant la substance à analyser ;
un tampon conjugué comprenant un corps conjugué primaire ayant une première liaison d'anticorps spécifiquement avec un premier épitope d'une substance à analyser et un premier indicateur auquel est lié le premier anticorps, et un corps conjugué secondaire ayant une troisième liaison d'anticorps spécifiquement avec le premier anticorps du corps conjugué primaire et un deuxième indicateur auquel est lié le troisième anticorps, dans lequel le corps conjugué primaire est disposé plus près d'un deuxième anticorps immobilisé que le corps conjugué secondaire, et dans lequel la particule du premier indicateur a une taille d'environ 10 nm à environ 20 nm et la particule du deuxième indicateur a une taille d'environ 20 nm à environ 60 nm,
dans lequel le deuxième indicateur est plus grand que le premier indicateur, de telle sorte que le deuxième corps conjugué atteigne le deuxième anticorps immobilisé après le corps conjugué primaire ;
une membrane comprenant un site de détection immobilisant en cet endroit le deuxième anticorps se liant spécifiquement au deuxième épitope de la substance à analyser auquel est lié le corps conjugué primaire, et un site de contrôle pour la détection d'erreur ; et
un tampon absorbant qui absorbe l'échantillon de liquide par un phénomène capillaire.

8. Kit immunochromatographique selon la revendication 6, dans lequel le connecteur est une albumine de sérum bovin (BSA).

9. Kit immunochromatographique selon les revendications 7 ou 8, dans lequel le tampon conjugué comporte un premier tampon conjugué contenant le corps conjugué primaire et un deuxième tampon conjugué contenant le corps conjugué secondaire, et est fabriqué de telle manière que le corps conjugué primaire et le corps conjugué secondaire n'entrent pas en contact l'un avec l'autre.

10. Kit immunochromatographique selon les revendications 7 ou 8, dans lequel les indicateurs sont des colloïdes d'or ou des points quantiques.

11. Kit immunochromatographique selon les revendications 7 ou 8, dans lequel la substance à analyser est choisie dans le groupe constitué d'une protéine antigène, d'un acide désoxyribonucléique (ADN), d'une hormone environnementale, d'une substance poison et d'une bactérie empoisonnant des aliments.
